# EUROPÄISCHE PATENTSCHRIFT

(11) **EP 2 735 390 B1**
(45) Veröffentlichungstag und Bekanntmachung des Hinweises auf die Patenterteilung: **10.10.2018**
(21) Anmeldenummer: 13194338.3
(22) Anmeldetag: 25.11.2013
(51) Int. Cl.: B22F 1/00, B22F 9/04, B82Y 30/00

(54) **KOHLENSTOFFFREIE SUSPENSION AUS GOLD- ODER METALNANOPARTIKELN AUS DER PLATINGRUPPE , VORRICHTUNG UND VERFAHREN ZU DEREN HERSTELLUNG.**
SUSPENSION OF NANOPARTICLES OF GOLD OR OF METAL FROM THE GROUP OF PLATIN, APPARATUS AND METHOD FOR THE MANUFACTURE THEREOF.
SUSPENSION DE NANOPARTICULES D'OR OU D'UN METAL DU GROUPE DU PLATINE, APPAREIL ET PROCÉDÉ POUR LA FABRICATION D'UNE TELLE SUSPENSION.

(30) Priorität: 23.11.2012 EP 12194129
(43) Veröffentlichungstag der Anmeldung: 28.05.2014
(73) Patentinhaber: Universität Duisburg-Essen, 45141 Essen (DE)
(72) Erfinder: Lau, Marcus, 45130 Essen (DE); Barcikowski, Stephan, 45359 Essen (DE)
(74) Vertreter: Taruttis, Stefan Georg

(56) Entgegenhaltungen:
- US-A1- 2006 068 026
- US-A1- 2011 303 050
- JEAN-PHILIPPE SYLVESTRE ET AL: "Surface Chemistry of Gold Nanoparticles Produced by Laser Ablation in Aqueous Media", THE JOURNAL OF PHYSICAL CHEMISTRY B, Bd. 108, Nr. 43, 10. Juni 2004 (2004-06-10), Seiten 16864-16869, XP055004486, ISSN: 1520-6106, DOI: 10.1021/jp047134+
- CHRISTOPH REHBOCK ET AL: "Size control of laser-fabricated surfactant-free gold nanoparticles with highly diluted electrolytes and their subsequent bioconjugation", PHYSICAL CHEMISTRY CHEMICAL PHYSICS, Bd. 15, Nr. 9, 3. Oktober 2012 (2012-10-03), Seite 3057, XP055105492, ISSN: 1463-9076, DOI: 10.1039/c2cp42641b
- A. V. KABASHIN ET AL: "Synthesis of colloidal nanoparticles during femtosecond laser ablation of gold in water", JOURNAL OF APPLIED PHYSICS, Bd. 94, Nr. 12, 15. Dezember 2003 (2003-12-15), Seite 7941, XP055105495, ISSN: 0021-8979, DOI: 10.1063/1.1626793
- Carlos J. Bueno-Alejo ET AL: "Ultraclean Derivatized Monodisperse Gold Nanoparticles through Laser Drop Ablation Customization of Polymorph Gold Nanostructures", Langmuir, vol. 28, no. 21, 29 May 2012 (2012-05-29), pages 8183-8189, XP055370960, US ISSN: 0743-7463, DOI: 10.1021/la3010689
- Itzel E. Santizo ET AL: "Intrinsic Chirality in Bare Gold Nanoclusters: The Au 34 - Case", Journal of Physical Chemistry C, vol. 112, no. 45, 13 November 2008 (2008-11-13), pages 17533-17539, XP055434564, US ISSN: 1932-7447, DOI: 10.1021/jp806080b
- Haijun Zhang ET AL: "Catalytically highly active top gold atom on palladium nanocluster", Nature Materials, vol. 11, no. 1, 23 October 2011 (2011-10-23), pages 49-52, XP055434566, GB ISSN: 1476-1122, DOI: 10.1038/nmat3143

## Beschreibung

Die vorliegende Offenbarung betrifft ein Verfahren zur Herstellung von kleinen Nanopartikeln und Atomclustern, bevorzugt aus Gold, die insbesondere einen Durchmesser von kleiner als 5 nm, bevorzugt kleiner 3 nm aufweisen und bevorzugt frei von organischen Kohlenstoffverbindungen sind, insbesondere kohlenwasserstofffrei sind, z.B. als einzige Kohlenstoffverbindung CO₂, insbesondere in Wasser gelöst, z.B. als Carbonatanionen, aufweisen und besonders bevorzugt kohlenstofffrei sind, in wässriger Zusammensetzung. Weiter betrifft die Erfindung die mit dem Verfahren erhältlichen Nanopartikel, die in Suspension vorliegen und gegen Agglomeration stabil sind, wobei die Suspension frei von organischen Kohlenstoffverbindungen ist, insbesondere kohlenwasserstofffrei, z.B. als einzige Kohlenstoffverbindung CO₂, insbesondere in Wasser gelöst, z.B. als Carbonatanionen, aufweist und besonders bevorzugt kohlenstofffrei ist. Insofern sind die erfindungsgemäß hergestellten Nanopartikel rein. In Suspension, die eine wässrige Suspension ist, können die Nanopartikel in Form von Clustern vorliegen, die bevorzugt Vollschalencluster sind, insbesondere aus einer definierten Anzahl Atomen, z.B. Au-Cluster aus 55 Atomen. Die Nanopartikel können im Anschluß an ihre Herstellung optional aneinander angelagert sein.

Weiter betrifft die Offenbarung eine Vorrichtung, die für das Herstellungsverfahren von Nanopartikeln angepasst ist.

Weiter werden Verbindungen und Beschichtungen der mit dem Verfahren erhältlichen Nanopartikel beschrieben, beispielsweise mit den Nanopartikeln verbundene organische Moleküle, insbesondere biologisch aktive Verbindungen, sowie mit den Nanopartikeln beschichtete anorganische Träger, Elektroden oder optische Bauteile, insbesondere optische Elemente, die eine Schicht aus den Nanopartikeln aufweisen. Solche Verbindungen werden z.B. durch Kontaktieren der Nanopartikel, die frei von organischen Kohlenstoffverbindungen sind, mit einem organischen Molekül erhalten. Die Nanopartikel bestehen bevorzugt aus einem Edelmetall, insbesondere aus Gold und/oder einem Platinmetall, z.B. Platin.

### Stand der Technik

Die US 2011/0303050 A1 beschreibt zur Detektion von Zyanid in Wasser die Erzeugung von ZnO₂-Nanopartikeln durch UV-Laserbestrahlung eines Zinkkörpers in Wasser mit einem Oxidationsmittel.

Die US2006/0068026 A1 beschreibt die elektrochemische Umsetzung einer Silberanode in reinem Wasser zu Silberkationen, die durch den Stromfluss zu metallischen Nanopartikeln in Suspension reduziert werden.

Die WO 2012/080458 A1 beschreibt ein Verfahren zur Herstellung metallischer Nanopartikel durch Laserablation, die anschließend mit wasserunlöslichen Mikropartikeln in Suspension gemischt werden, um die Nanopartikel auf den Mikropartikeln adsorbieren zu lassen. Bei dem Verfahren soll der Oxidationsgrad der Nanopartikel durch Einsatz eines organischen Lösungsmittels, insbesondere Aceton, Ethanol oder Isopropanol beeinflusst werden, oder durch Einsatz von Liganden, beispielsweise durch Natriumzitrat.

Die WO 2010/087869 A1 beschreibt die Herstellung von Nanopartikeln durch ultrakurze gepulste Laserablation in Flüssigkeiten, die sich dadurch auszeichnet, dass die Flüssigkeit, insbesondere Wasser, keine chemischen Stabilisatoren enthalten soll, wobei die Stabilität der Suspension der Nanopartikel durch Flüssigkeitsbewegung erhöht werden kann.

Die WO 2010/007117 beschreibt ein Verfahren zur Herstellung von Konjugaten aus metallischen Nanopartikeln mit einem organischen Bestandteil durch Bestrahlen eines Metallkörpers mit einem Laserstrahl, wobei ein den Metallkörper umgebendes Trägerfluid, das mit einer Vorläuferverbindung des organischen Bestandteils versetzt ist, bewegt wird.

Lopez-Sanchez et al., Nature Chemistry 1-6 (2011) beschreiben die Herstellung von metallischen Nanopartikeln in reduzierendem Medium mit Polyvinylalkohol (PVA) als Stabilisator, der von geträgerten Nanopartikeln durch Erwärmen auf bis zu 400°C oder Waschen mit heißem Wasser zumindest teilweise entfernt wurde.

Werner et al., J. Phys. Chem. C 16801-16808 (2008) beschreiben für die Erzeugung von suspendierten Goldnanopartikeln durch Laserbestrahlung von Goldflocken einer Dicke von 0,1-0,2µm zu Nanopartikeln mit <3nm nur in Anwesenheit von organischem Lösungsmittel, das unter Laserbestrahlung zerfällt. Bei Laserbestrahlung der Goldflocken in Wasser führt ein zuvor erzeugter Gehalt des Wassers an O₂ zur schnellen Agglomeration und Ausfällung erzeugter Nanopartikel, während die vorherige Begasung mit Argon oder ein Gehalt an Zitrat dies verhindert. Nicht plasmonresonante Partikel konnten mittels Laserbestrahlung von Goldflocken nur bei Zugabe des organischen Stabilisators Dodecanthiol erzeugt werden.

Sylvestre et al., J. Phys. Chem. B, 16864-16869 (2004) beschreiben, dass die Laserbestrahlung eines Goldstabs in reinem Wasser zu Partikeln von 1-250nm mit einer mittleren Größe von 40nm führt und in NaCl oder KCl oder Propylamin, je 10mM, oder in NaOH die Größenverteilung abnimmt und die mittlere Partikelgröße auf 5,5-8nm sinkt.

Nichols et al., J. Appl. Phys. 114911 (2006) analysieren den Ablauf der Erzeugung von Nanopartikeln bei Laserbestrahlung einer Platinplatte in Wasser, insbesondere die Kraterbildung auf der Platinplatte und deren Abhängigkeit von der Laserstrahlung.

Bueno-Alejo et al, Langmuir 8183-8189 (2012) beschreiben eine Methode zur chemischen Synthese von Goldnanopartikeln durch Reduktion von AuCl4- mit H2O2 unter Bestrahlung mit UV-Licht, welche anschließend durch Laserbestrahlung in einem Flüssigkeitstropfen zur Größenreduktion nachbestrahlt werden.

### Aufgabe der Erfindung

Der Erfindung stellt sich die Aufgabe, ein alternatives Verfahren zur Herstellung von Nanopartikeln, die Cluster sein können, bereitzustellen, die in Medium ohne organische Kohlenstoffverbindung, z.B. in einem kohlenstofffreien Medium wie Wasser eine stabile Suspension bilden..

### Allgemeine Beschreibung der Erfindung

Die Erfindung löst die Aufgabe mit den Merkmalen der Ansprüche und stellt ein Verfahren zur Herstellung von Nanopartikeln bereit, die durch das Verfahren hergestellte Suspension der Nanopartikel, sowie eine Vorrichtung, die zur Herstellung der Nanopartikel eingerichtet ist. Das Verfahren zeichnet sich dadurch aus, dass die Nanopartikel rein, frei von organischen Kohlenstoffverbindungen, bevorzugt kohlenstofffrei, und kontinuierlich erhalten werden. Das Verfahren kann vollständig oder teilweise kontinuierlich ausgeführt werden und ist daher auf einfache Weise skalierbar. Die Nanopartikel, die mit dem Verfahren erhältlich sind, zeichnen sich dadurch aus, dass sie ohne einen organischen Liganden, insbesondere ohne organische Verbindung als Stabilisator, in Suspension vorliegen und insbesondere bevorzugt als Suspension gegen Agglomeration stabil sind, wobei das Medium mit den darin suspendierten Partikeln frei von organischen Kohlenstoffverbindungen, insbesondere bevorzugt kohlenstofffrei ist. Generell werden unter den Nanopartikeln auch deren Cluster aus Metallatomen, insbesondere Vollschalencluster gefasst.

Das Verfahren zur Herstellung von Nanopartikeln sieht vor, mit einer ersten Laserstrahlung, die insbesondere gepulst, bevorzugt eine Kurzpulsstrahlung ist, aus einem metallhaltigen Körper erste Partikel zu erzeugen, wobei der metallhaltige Körper in einem wässrigen Medium angeordnet ist. Der metallhaltige Körper kann insbesondere ein Metallkörper (Oxidationsstufe 0) sein, wahlweise in Drahtform oder ein Pulver oder Mikropulver (Korngröße z.B. von 1 µm bis 500µm) das in dem Medium suspendiert sein kann. Als Metall ist ein Metall der Platingruppe, nachfolgend auch Platinmetall genannt, z.B. Platin, und/ oder Gold enthalten; bevorzugt besteht der metallhaltige Körper aus Gold, bevorzugt mit einer Reinheit von größer als 99,9%, optional aus einer Legierung von zwei oder mehr Metallen der Platingruppe, z.B. AuPt.

Die erste Laserstrahlung ist vorzugsweise eine Kurzpulsstrahlung mit hoher Pulsenergie, vorzugsweise mit einer Pulsenergie von 1 bis 100 mJ, insbesondere mit Repetitionsraten von 10 Hz bis 500 kHz, insbesondere 0,1 bis 10 kHz, z.B. mit einer Pulsdauer von 100 ns, bevorzugt von maximal 10 ns, bevorzugter von maximal 10 ps, bei einer Wellenlänge von größer 330 nm und maximal 1500 nm, insbesondere im Bereich von 532 bis 1064 nm, bevorzugt 1064 nm. Die Zeitdauer der Bestrahlung des metallhaltigen Körpers beträgt vorzugsweise ca. 5 bis 10 min, z.B. 10 min je 100 mL Medium, bei einer Pulsenergie von ca. 80 mJ bei 10 ns Pulsdauer oder ca. 100 µJ bei 10 ps Pulsdauer.

Generell bevorzugt wird das Medium während der Bestrahlung des metallhaltigen Körpers mit der ersten Laserstrahlung bewegt, beispielsweise durch Rühren oder Überströmen des metallhaltigen Körpers mit dem Medium. Alternativ oder zusätzlich wird die erste Laserstrahlung relativ zum metallhaltigen Körper bewegt, z.B. durch Bewegen des Laserstrahls oder durch Bewegen des metallischen Körpers.

Erfindungsgemäß weist das Medium bevorzugt auf oder besteht aus Wasser, das frei ist von organischen Kohlenstoffverbindungen, insbesondere als einzige Kohlenstoffverbindung CO₂ enthält und bevorzugter kohlenstofffrei ist, mit einem Gehalt an zumindest einem anorganischen Oxidationsmittel. Das anorganische Oxidationsmittel kann z.B. ausgewählt sein aus H₂O₂, Ozon, hypohalogenigen und halogenigen Oxidationsmitteln und sämtlichen nichtkohlenstoffhaltigen oxidativ wirkenden Verbindungen, z.B. Derivate aus Sauerwasserstoffen, Stickoxide, Antimonsäure, arsenige Säure, Arsensäure, Borsäure, chlorige Säure, bromige Säure, Chlorsäure, Chromsäure, Cyansäure, Dichromsäure, Dischwefelsäure, hypochlorige Säure, hypobromige Säure, hypoiodige Säure, iodige Säure, Iodsäure, Isocyansäure, Kohlensäure, Metakieselsäure, Molybdänsäure, Orthodikieselsäure, Orthokieselsäure, Perbromsäure, Perchlorsäure, Periodsäure (Orthoperiodsäure), Peroxodischwefelsäure, Peroxosalpetersäure, Phosphorsäure, Salpetersäure, salpetrige Säure, Schwefelsäure, schweflige Säure, Tellursäure, Thioschwefelsäure, Wolframsäure sowie deren Salze, hypofluorige Säure, Hypofluorite, Chlorsauerstoffsäuren, Hypochlorite, Chlorite, Chlorate, Perchlorate, Bromsauerstoffsäuren, Hypobromite, bromige Säure, Bromite, Bromsäure, Bromate, Perbromsäure, Perbromate, Iodsauerstoffsäuren, Hypoiodite, Iodite, Iodsäure, Iodate, ortho-Periodsäure, Periodate, meta-Periodsäure und Mischungen dieser.

Dabei weist das Oxidationsmittel ein Redoxpotential in dem wässrigen Medium größer als das oxidierter Formen von Gold oder eines Metalls der Platingruppe auf, die durch die Laserstrahlung entstehen, insbesondere größer als das Redoxpotential von Au⁺ und/oder Au³⁺ in dem Medium. Bevorzugt weist das Oxidationsmittel in dem wässrigen Medium ein um zumindest 0,05V, bevorzugt zumindest 0,09V, bevorzugter zumindest 0,15V oder zumindest 0,2V, bevorzugter zumindest 0,3 bis 0,38V größeres Redoxpotential als eine oxidierte Form von Gold bzw. eines Platinmetalls auf. Es wird gegenwärtig angenommen, dass die Wirkung des anorganischen Oxidationsmittels darauf beruht, die durch die (zweite) Laserbestrahlung, die auf die ersten Partikel gerichtet ist, erzeugten Nanopartikel zu erhalten bzw. zu konservieren. Entsprechend kommt es für diese Wirkung darauf an, dass das anorganische Oxidationsmittel ein größeres Redoxpotential hat als es die oxidierte Form des Golds bzw. des Metalls der Platingruppe in der Größe der Nanopartikel aufweist, insbesondere in einer Größe von 1 bis 5nm. Die Abhängigkeit des Redoxpotentials der oxidierten Form von Gold bzw. des Metalls der Platingruppe von deren Partikelgröße ist generell bekannt. Dabei kann das Redoxpotential der oxidierten Form von Gold bzw. des Metalls der Platingruppe, die optional dessen höhere oder niedrigere Oxidationsstufe ist, bei der Größe der Nanopartikel kleiner sein als das Redoxpotential größerer Partikel von Gold bzw. Metalls der Platingruppe in der oxidierten Form.

Weiter bevorzugt weist das anorganische Oxidationsmittel, insbesondere wenn es ein in dem wässrigen Medium vorliegendes Gas ist, z.B. verteiltes oder gelöstes Gas, ein Redoxpotential auf, das zumindest um den Betrag der Überspannung der Nanopartikel größer als das Redoxpotential der oxidierten Form von Gold bzw. Metall der Platingruppe ist, die optional in der Größe der Nanopartikel vorliegen.

Das Redoxpotential der oxidierten Form von Gold bzw. des Metalls der Platingruppe in der Größe der Nanopartikel in dem wässrigen Medium und/oder die Überspannung dieser Nanopartikel kann vorbestimmt sein, z.B. für Nanopartikel, die durch das erfindungsgemäße Verfahren mit H₂O₂ in Wasser erzeugt wurden, insbesondere wie in Beispiel 1 beschrieben ist.

Da das Redoxpotential oxidierter Formen von Gold bzw. des Metalls der Platingruppe vom pH des wässrigen Mediums abhängen kann, weist das wässrige Medium optional eine von organischem Kohlenstoff freie Puffersubstanz auf, z.B. Carbonat, Phosphat, optional in Kombination mit einer weiteren Säure oder Base, die den pH des wässrigen Mediums auf einen Wert einstellen, bei dem das Redoxpotential des Oxidationsmittels über dem der oxidierten Formen des Golds bzw. des Metalls der Platingruppe liegt.

Redoxpotentiale unter Standardbedingungen

| Bezeichnung | Formel | Redoxpotential (bei pH 0 gegen Wasserstoffelektrode) | pH 7 | pH 14 |
|---|---|---|---|---|
| Wasser | H₂O | +1.23 V | +0,82 V | -0,828 V |
| Sauerstoff | O₂ | +1,23 V | + 0,82 V | |
| Gold | Au⁺ | +1,69 V | | +1 V |
| Gold | Au³⁺ | +1,5 V | | +0,535 V |
| Wasserstoffperoxid | H₂O₂ | +1,78 V | | |
| Ozon | O₃ | +2,07 V | | |
| atomarer Sauerstoff | O | +2,42 V | | |

Weiter bevorzugt weist das wässrige Medium das Oxidationsmittel in einer molaren Konzentration des zumindest 1.000-fachen, bevorzugter zumindest 2.000-fachen, noch bevorzugter zumindest 10.000-fachen, weiter bevorzugt der zumindest 100.000-fachen oder zumindest 200.000-fachen berechneten Konzentration von Nanopartikeln mit einer Größe von 2nm auf. Die Massenkonzentration von Nanopartikeln einer Größe von 2nm ergibt sich aus der Massenkonzentration der im Verfahren eingesetzten ersten Partikel in mg/L.

Die ersten Partikel, die durch eine erste Laserbestrahlung eines Metalls in Wasser erzeugt werden, haben z.B. für zumindest 90%, bevorzugter zumindest 95% der Partikel, bevorzugt eine Größe von zumindest 10 nm bis maximal 250 nm, bevorzugter bis maximal 50nm, noch bevorzugt von 10 bis 35nm, z.B. mit einer mittleren Größe von ca. 18 bis 22 nm.

Das Verfahren sieht vor, die durch das Aufbringen der ersten Laserstrahlung auf den metallhaltigen Körper erzeugten ersten Partikel, die in dem Medium enthalten sind, mit einer gepulsten Laserstrahlung zu bestrahlen, die auch als zweite Laserstrahlung bzw. als zweite gepulste Laserstrahlung bezeichnet wird, um die Nanopartikel zu erzeugen.
Dabei wird bevorzugt ein Strom des Mediums, das frei ist von organischen Kohlenstoffverbindungen, insbesondere als einzige Kohlenstoffverbindung CO2 enthält und bevorzugter kohlenstofffrei ist, wobei das Medium ein anorganisches Oxidationsmittel enthält, mit den darin suspendierten ersten Partikeln erzeugt und dieser Strom wird mit der zweiten gepulsten Laserstrahlung bestrahlt. Vorzugsweise ist der Strom des Mediums ein freier Strom, beispielsweise ein Strom, der ohne Kontakt mit einer festen Oberfläche zumindest in dem Abschnitt vorliegt, der von der zweiten gepulsten Laserstrahlung bestrahlt wird. Insbesondere ist der Strom des Mediums zumindest in dem von der zweiten gepulsten Laserstrahlung bestrahlten Abschnitt nicht in einem Rohr geführt, sondern z.B. in der Form als kontinuierlicher oder tropfenförmiger Flüssigkeitsstrom in einem gasgefüllten Raum. Der Strom des Mediums mit den darin suspendierten ersten Partikeln ermöglicht vorteilhafter Weise die Bestrahlung mit hoher Fluenz.

Alternativ können die in dem Medium mit anorganischem Oxidationsmittel enthaltenen ersten Partikel mit der zweiten Laserstrahlung bestrahlt werden, während das Medium unbewegt in einem Behälter ist. Bei dieser Verfahrensführung ist bevorzugt, dass die zweite Laserstrahlung relativ zum Medium bewegt wird, insbesondere durch Bewegen des Mediums, z.B. durch einen Rührer oder mittels Umwälzens.

Das anorganische Oxidationsmittel kann im Medium vorgelegt sein, in dem der metallhaltige Körper während der Bestrahlung mit erster Laserstrahlung angeordnet ist und/oder während der Bestrahlung mit erster Laserstrahlung kontinuierlich oder satzweise zugegeben werden. Alternativ oder zusätzlich kann das anorganische Oxidationsmittel dem Medium vor Bestrahlen mit der zweiten Laserstrahlung kontinuierlich oder satzweise zugesetzt werden.

Optional kann das Medium anorganische Ionen aufweisen, z.B. Alkaliionen, Erdalkaliionen, Halogenionen und/oder anorganische Anionen, z.B. Phosphat, Sulfat; bevorzugt besteht das Medium aus Wasser und zumindest einem anorganischen Oxidationsmittel und ist frei von anorganischen Ionen.

Optional wird der pH-Wert des Mediums vor der Bestrahlung mit erster Laserstrahlung und/oder vor der Bestrahlung mit zweiter Laserstrahlung auf einen Wert von 3 bis 6,5 eingestellt.

Bevorzugt ist die zweite Laserstrahlung auf den Strom des Mediums mit den darin suspendierten ersten Partikeln fokussiert, wobei bevorzugt der Durchmesser des Stroms des Mediums mit den darin suspendierten ersten Partikeln maximal so groß ist, wie der Fokus der zweiten gepulsten Laserstrahlung. Bevorzugt liegt die Wellenlänge der zweiten Laserstrahlung in dem Bereich des Extinktionsmaximums der in dem Medium suspendierten ersten Partikel, z.B. im grünen Bereich, insbesondere bei 510 bis 540 nm.

Die zweite gepulste Laserstrahlung kann die Eigenschaften haben, wie sie mit Bezug auf die erste Laserstrahlung beschrieben sind, insbesondere bei einer Repetitionsrate von zumindest 10 Hz, bevorzugter von zumindest 100 Hz, bevorzugter von zumindest 1000 Hz, insbesondere für Pulsdauern von 1 bis 100 ns, bei 1 bis 500 kHz für Pulsdauern von 0,5 bis 100 ps, bevorzugt 1 bis 20 ps.

Ein Grund dafür, dass zumindest in dem Abschnitt, in dem das Medium mit den darin suspendierten ersten Partikeln von der zweiten gepulsten Laserstrahlung bestrahlt wird, bevorzugt in einem freien Strom geführt wird, ist die Vermeidung von Wechselwirkungen der Laserstrahlung und/oder der erzeugten Partikel mit einem Wandmaterial. Weiterhin sind in dieser Ausführungsform hohe Laserfluenzen, beispielsweise mit einer Laserfluenz von 0,1 bis 25 J/cm², bevorzugt >1 J/cm² möglich, ohne dass eine entstehende Ultraschallwelle, Kavitationsblase, Stoßwelle, Laserabsorption oder thermische Effekte ein Wandmaterial, das den Strom des Mediums kontaktiert, insbesondere eine Kapillare, belasten würde. Dieses Verfahren und die dafür verwendete Vorrichtung erlauben eine intensive Bestrahlung der Partikel und insbesondere die Herstellung von Nanopartikeln mit einer engen Partikelgrößenverteilung, die für die Stabilität der Nanopartikel in Suspension vorteilhaft ist.

Das Medium, in dem der metallhaltige Körper bestehend aus Gold und/oder aus einem Metall der Platingruppe während des Aufbringens der ersten gepulsten Laserstrahlung angeordnet ist, kann von dem Medium abweichen, aus dem ein Strom erzeugt wird, wobei in diesem Medium die ersten Partikel suspendiert sind. Z.B. kann das Medium, in dem der metallhaltige Körper bestehend aus Gold und/oder aus einem Metall der Platingruppe während der Bestrahlung mit der ersten gepulsten Laserstrahlung angeordnet ist, ein wässriges Medium sein, insbesondere frei sein von einem anorganischen Oxidationsmittel, insbesondere aus reinem Wasser bestehen, während das Medium, aus dem der Strom erzeugt wird, von dem zumindest ein Abschnitt mit der zweiten gepulsten Laserstrahlung bestrahlt wird, das anorganische Oxidationsmittel enthält, insbesondere aus Wasser und zumindest einem anorganischen Oxidationsmittel besteht. Bevorzugt ist der pH-Wert und der Gehalt an gelöstem Gas des Mediums, in dem der metallhaltige Körper während des Aufbringens der ersten gepulsten Laserstrahlung angeordnet ist, eingestellt, z.B. durch Zugabe anorganischer Säure bzw. Base, bzw. durch Begasen oder Ausgasen.

Bevorzugt weist der Strom in dem Abschnitt, in dem er von der zweiten Laserstrahlung bestrahlt wird, einen Durchmesser von maximal 3 mm auf, insbesondere von 1 bis 3 mm, z. B. von 1,2 mm, wobei z.B. der zweite Laserstrahl einen Rohstrahldurchmesser von maximal 8 mm, bevorzugt maximal 6 mm aufweisen kann. Weiter bevorzugt ist der Strom vertikal nach unten gerichtet, bzw. ein frei vertikal fallender Strom des Mediums mit darin suspendierten ersten Partikeln. Bevorzugt ist der zweite Laserstrahl senkrecht auf den Strom dieses Mediums gerichtet.

Bevorzugt wird der Strom dadurch erzeugt, dass das Medium mit den darin suspendierten ersten Partikeln durch eine Düse austreten gelassen wird, deren Längsachse bevorzugt vertikal angeordnet ist. Die Düse ist bevorzugt kontinuierlich mit dem Medium mit den darin suspendierten ersten Partikeln beaufschlagt, beispielsweise aus einem Vorratsbehälter, bevorzugt unmittelbar im Anschluss an das Aufbringen der ersten gepulsten Laserstrahlung auf den ersten metallhaltigen Körper, sodass das Medium mit den darin suspendierten ersten Partikeln bevorzugt innerhalb von 60 min, bevorzugter innerhalb von 10 min, bevorzugter innerhalb 1 min zum Erzeugen des Stroms verwendet wird, beispielsweise durch Zuführung zu einer Düse.

In Alternative zum freien Strom kann das Medium, das die ersten Partikel enthält, während es mit der gepulsten Laserstrahlung bestrahlt wird, in einem Behälter enthalten sein, ohne oder mit Flüssigkeitsbewegung. Ein solcher Behälter kann geschlossen oder offen sein und das Medium mit den ersten Partikeln enthalten oder von dem Medium mit den ersten Partikeln durchströmt werden. Ein Behälter kann z.B. eine Durchflusskammer bzw. ein Rohr sein, wobei der gepulste Laser einen Volumenabschnitt durchstrahlt, wobei der Laser insbesondere eingerichtet ist, jedes Volumenelement des Mediums mit den ersten Partikeln zumindest oder genau einmal zu durchstrahlen.

Weiter bevorzugt wird dem Medium nach Bestrahlung mit dem zweiten Laserstrahl anorganisches Oxidationsmittel zugesetzt, z.B. in Abhängigkeit von der Konzentration und/oder Größe erzeugter Nanopartikel gesteuert zugesetzt. Das im Anschluß zugesetzte anorganische Oxidationsmittel kann dasjenige sein, das das Medium vor Bestrahlung mit dem zweiten Laserstrahl enthält, oder ein anderes anorganisches Oxidationsmittel.

Die mit dem Verfahren erzeugten Nanopartikel weisen eine maximale Größe von 5 nm auf, mit einer mittleren Größe von 2 bis 3 nm, insbesondere von 2,5 nm. Dabei weisen zumindest 50%, bevorzugter zumindest 75%, insbesondere bevorzugt zumindest 90% der Nanopartikel eine solche mittlere Größe auf. Bei Vorliegen dieser Nanopartikel in einem Medium in Suspension, die aus dem Medium und diesen Nanopartikeln besteht und daher insbesondere keine weiteren Partikel, z.B. keine ersten Partikel enthält, sind die erfindungsgemäß hergestellten Nanopartikel bevorzugt nicht oberflächenplasmonresonant, z.B. sind erfindungsgemäß hergestellte Nanopartikel aus Gold nicht oberflächenplasmonresonant bei Bestrahlung mit einer Wellenlänge von 520 nm. Nanopartikel, die Cluster sind, können Vollschalencluster wie M55, M309, M561, insbesondere Au55 oder Pt309 sein.

Es hat sich gezeigt, dass Bestrahlen des Stroms aus Medium, dem ein anorganisches Oxidationsmittel zugesetzt ist, mit darin suspendierten ersten Partikeln mit einer gepulsten Laserstrahlung, die für die Zwecke der Erfindung auch als zweite gepulste Laserstrahlung bezeichnet wird, Nanopartikel dieser Größe erzeugt.

Überraschenderweise hat sich gezeigt, dass die Anwesenheit eines anorganischen Oxidationsmittels in dem Medium, das erste, durch Laserbestrahlung eines metallhaltigen Körpers erzeugte Partikel enthält, zur Stabilität der durch Bestrahlen des Stroms mit einer zweiten gepulsten Laserstrahlung zur Erzeugung einer stabilen Zusammensetzung bzw. stabilen Verteilung der Nanopartikel in dem Medium führt. Entsprechend liegt ein besonderer Vorteil des erfindungsgemäßen Verfahrens und der damit erhältlichen Nanopartikel darin, dass die Zusammensetzung bzw. das Medium, in dem die Nanopartikel enthalten sind, kein Stabilisationsmittel, insbesondere keine organischen Verbindungen zu enthalten braucht. Die Stabilität der Suspension der Nanopartikel gegen Anlagerung wird gegenwärtig auf die zumindest teilweise oberflächliche, bevorzugt vollständige oberflächliche Oxidation der Nanopartikel zurückgeführt, bzw. darauf, dass die Nanopartikel in der Suspension elektrostatisch stabilisiert sind.

Dadurch, dass die erzeugten Nanopartikel ohne organische Kohlenstoffverbindung bzw. kohlenstofffrei in dem Medium stabil sind, können sie ohne Abtrennung einer Kohlenstoffverbindung mit einer zugesetzten Substanz reagieren und beispielsweise eine Komplexverbindung eingehen, chemisorbieren oder eine Beschichtung bilden, die insbesondere aus den Nanopartikeln besteht, insbesondere frei von Kohlenstoff ist. Solche Komplexverbindungen mit Nanopartikeln oder von organischen Kohlenstoffverbindungen freie Beschichtungen aus Nanopartikeln, insbesondere kohlenstofffreie Beschichtungen aus Nanopartikeln weisen eine signifikante katalytische Aktivität auf. Für die Beschichtungen aus Nanopartikeln wird die katalytische Aktivität auf die große Oberfläche der Nanopartikel und/oder auf die Interaktion der Nanopartikel mit dem Substrat zurückgeführt und darauf, dass die Oberfläche der Nanopartikel nicht von Kohlenstoffverbindungen belegt ist.

Entsprechend enthält das Verfahren bevorzugt den Schritt, anschließend die Nanopartikel in dem Medium mit einer Substanz zu kontaktieren, die beispielsweise ein biologisches Molekül ist, insbesondere ausgewählt aus Nukleinsäure, beispielsweise einem Oligonukleotid, einem Protein, Peptid, einem Glycosid und/oder einem Lipid. Bevorzugt ist das Protein, das mit den Nanopartikeln kontaktiert ist und eine Verbindung damit bildet ein spezifisches Bindemolekül, z.B. ein Antikörper. Das biologische Molekül weist vorzugsweise eine Thiolgruppe oder Disulfidgruppe auf. Alternativ oder zusätzlich kann die Substanz ein Träger, insbesondere ein anorganischer Träger sein, sodass die Nanopartikel auf diesem Träger abgeschieden werden und den Träger beschichten. Dazu ist bevorzugt, dass die Nanopartikel elektrophoretisch auf dem anorganischen Träger abgeschieden werden, um eine oberflächliche Beschichtung zu bilden. Ein solcher anorganischer Träger ist insbesondere ein Katalysatorträger, beispielsweise ein Titanoxid, sodass beispielsweise die Nanopartikel eine katalytisch aktive Schicht bilden, oder der anorganische Träger kann eine Elektrode sein, auf dem die abgeschiedenen Nanopartikel eine Beschichtung bilden. Eine Elektrode, die eine Beschichtung aus den Nanopartikeln aufweist, ist insbesondere zur Verwendung als elektrophysiologische Elektrode geeignet. In diesen Ausführungsformen liegt ein Vorteil der erfindungsgemäßen Nanopartikel darin, dass diese ein Fluoreszenzsignal bei Bestrahlung mit einer Anregungswellenlänge erzeugen, dass von einer an die Nanopartikel gebundenen Substanz beeinflusst ist, bzw. für die gebundene Substanz spezifisch ist.

Bevorzugt werden die Nanopartikel direkt elektrochemisch mit dem Träger gekoppelt, z.B. durch Chemi- oder Physisorption. Eine direkte elektrochemische Kopplung der Nanopartikel mit einer Substanz, die insbesondere ein biologisches Molekül ist, oder mit einem Träger erlaubt eine Orbitalkopplung, die in Elektrochemolumineszenz oder Fluoreszenz resultieren. Die Abwesenheit von organischen Kohlenstoffverbindungen, bevorzugt von Kohlenstoff, von den eingesetzten Nanopartikeln weist eine mit dem Nanopartikel kontaktierte bzw. gebundene Substanz keine weiteren organischen Kohlenstoffverbindungen auf bzw. eine Beschichtung aus den Nanopartikeln weist keine organischen Kohlenstoffverbindungen, die die Wechselwirkung der Nanopartikel mit der Substanz bzw. dem Träger beeinflussen oder die die Reaktion der am Nanopartikel gebundenen Substanz oder der Beschichtung auf dem Träger beeinflussen. Daher können aus den Nanopartikeln Verbindungen mit Substanzen oder Beschichtungen auf Trägern erzeugt werden, deren Eigenschaften, z.B. Reaktions- oder Bindeeigenschaften, nicht durch organische Kohlenstoffverbindungen beeinträchtigt werden.

Weiterhin kann die Substanz bzw. der Träger ein optisch aktiver Träger sein, beispielsweise ein optisch transparenter Träger, insbesondere aus Glas oder Kunststoff, sodass die Abscheidung der erfindungsgemäß hergestellten Nanopartikel auf dem Träger zur optisch aktiven Beschichtung führt. In dieser Ausführungsform führt das Verfahren zur Herstellung eines optischen Elements, da die Beschichtung der erfindungsgemäß hergestellten Nanopartikel auf dem Träger eine optisch aktive Schicht ist, die z.B. bei Lichteinfall einer vorbestimmten Wellenlänge die Lichtdurchlässigkeit der Beschichtung und damit des optischen Elements bei Erreichen oder Überschreiten einer bestimmten eingestrahlten Fluenzintensität schnell verringert, insbesondere um zumindest 50%, bevorzugter um zumindest 90%, bevorzugter um zumindest 99% zu reduzieren. Ein solches optisches Element kann z.B. als optischer Schalter, insbesondere für Laserstrahlung, die optional gepulst sein kann, insbesondere mit einer Wellenlänge von 350-1064 nm dienen, oder für sichtbares Licht. Ein solches optisches Element kann wegen der sehr schnellen Verringerung einfallender Strahlung als optische Schutzeinrichtung verwendet werden.

Die Verfahren, die einen Schritt des Kontaktierens der Nanopartikel mit einer Substanz, z.B. einem biologischen Molekül oder einem Träger enthalten, weisen vorteilhafter Weise keinen vorherigen Schritt zur Entfernung einer organischen Verbindung aus der Suspension der Nanopartikel auf. So können bei erfindungsgemäßen Verfahren Nanopartikel auf Trägern z.B. ohne chemische oder thermische Schritte, insbesondere ohne das Kalzinieren angeordnet werden, so dass die Schritte, die z.B. bei einem Gehalt eines Nanopartikelpräparats an Kohlenstoff zur Entfernung organischer Verbindungen notwendig sind, erfindungsgemäß nicht erforderlich sind.

H₂O₂ oder Ozon als anorganisches Oxidationsmittel sind dadurch vorteilhaft, dass sie durch Bestrahlung mit Licht, durch Druckabsenkung und/oder Temperaturerhöhung aus Wasser, das das Medium bildet, entfernt werden können. Entsprechend kann das Verfahren optional den Schritt aufweisen, im Anschluss an die Bestrahlung des Stroms des Mediums mit den darin suspendierten ersten Partikeln, wobei das Medium das anorganische Oxidationsmittel enthält, das Medium mit den darin enthaltenen Nanopartikeln zu bestrahlen, um insbesondere das anorganische Oxidationsmittel, das aus H₂O₂ oder Ozon besteht, zumindest anteilig zu entfernen. Bevorzugt wird das Medium dabei unter Unterdruck gesetzt und/oder mit Strahlung einer Wellenlänge bestrahlt, bei der das anorganische Oxidationsmittel zersetzt wird, z.B. im UV-Bereich, sodass das anorganische Oxidationsmittel bzw. dessen Zersetzungsprodukte gasförmig austreten können.

Die Vorrichtung ist insbesondere zur Durchführung der Schritte des Verfahrens eingerichtet.

### Genaue Beschreibung der Erfindung

Die Erfindung wird nun genauer anhand von Beispielen und mit Bezug auf die Figuren beschrieben, die in
Fig. 1 schematisch den Aufbau einer erfindungsgemäßen Vorrichtung,
Fig. 2 ein Photospektrum erfindungsgemäß hergestellter Nanopartikel in Wasser mit H₂O₂,
Fig. 3a eine TEM-Aufnahme erfindungsgemäßer Nanopartikel,
Fig. 3b die per TEM gemessene Größenverteilung erfindungsgemäß hergestellter Nanopartikel,
Fig. 3c eine graphische Darstellung der mit den Daten aus Fig. 3b bestimmten Sphärizität erfindungsgemäßer Nanopartikel,
Fig. 3d eine graphische Darstellung der Summenverteilung der Größen erfindungsgemäßer Nanopartikel,
Fig. 3e eine graphische Darstellung der per Scheibenzentrifuge gemessenen Größen erfindungsgemäßer Nanopartikel,
Fig. 4 in a) die relative Häufigkeit von Größen erfindungsgemäß hergestellter Partikel und deren elektronenmikroskopische Abbildung und in b) die relative Häufigkeit von Größen erster Partikel und deren elektronenmikroskopische Abbildung,
Fig. 5 eine rasterelektronenmikroskopische Aufnahme eines Trägers,
Fig. 6 eine rasterelektronenmikroskopische Aufnahme eines Trägers mit darauf angeordneten erfindungsgemäßen Nanopartikeln,
Fig. 7 eine rasterelektronenmikroskopische Aufnahme eines Trägers mit darauf angeordneten erfindungsgemäßen Nanopartikeln,
Fig. 8 das Ergebnis einer EDX-Analyse erfindungsgemäßer Nanopartikel auf einem Träger und
Figur 9 die berechnete molare Konzentration von Oxidationsmitteln und die berechnete Oberflächenatomanzahl von Nanopartikeln aus Gold bei einer Konzentration von 20 mg/L zeigen.

### Beispiel 1: Herstellung von Nanopartikeln aus Gold

Als metallhaltiger Körper wurde 99,99 %-iges Gold in hochreinem Wasser mit einem Gehalt an 10 Gew.-% H₂O₂ angeordnet und 10 ns Laserimpulsen bei einer Wellenlänge von 1064 nm mit einer maximalen Energie von 80 mJ pro Puls, Strahldurchmesser 6 mm bei einem Abstand von etwa 100 mm von der Linse (Brennweite 100 mm) und Metallkörper bei einer Pulswiederholungsrate von 100 Hz bestrahlt. Die Schichthöhe des Mediums zwischen Metallkörper und Laser betrug etwa 1 cm, der pH-Wert des Wassers ca. 3 bis 5.

Es konnte beobachtet werden, dass sich am Metallkörper eine Rotfärbung in der Flüssigkeit ausbreitete, wie dies für Goldnanopartikel bekannt ist. Es zeigte sich eine Plasmonresonanz (erhöhte Extinktion) bei 520 nm. Diese ersten Partikel agglomerierten zu hydrodynamischen Durchmessern von ca. 50 nm, gemessen durch dynamische Laserlichtstreuung und Messungen in einer Scheibenzentrifuge.

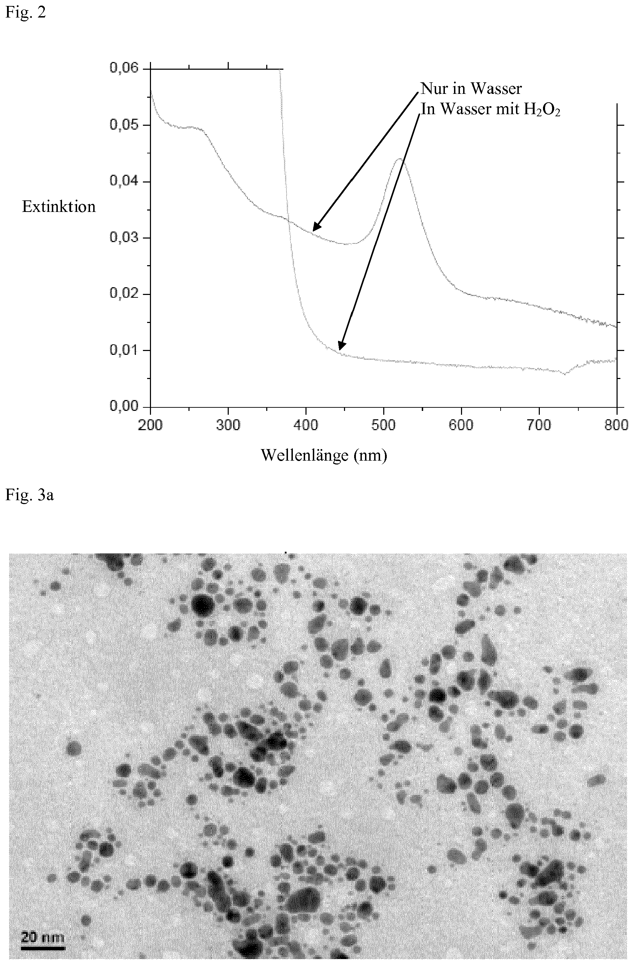

Das Medium mit den so hergestellten ersten Partikeln wurde unter Schwerkrafteinfluss oder mittels einer Pumpe durch eine Düse austreten gelassen, die innerhalb eines größeren Glaskörpers als Schutzbehälter angeordnet war. Die Düse war vertikal nach unten gerichtet und das Medium mit den darin suspendierten ersten Partikeln erzeugte einen frei fallenden Strom von ca. 1,2 mm Durchmesser. Ein zweiter Laser, der dieselben Strahleigenschaften wie der zuvor eingesetzte Laser jedoch die frequenzverdoppelte Wellenlänge von 532 nm hatte, wurde auf den Strom des Mediums fokussiert, sodass der fokussierte Laser den Strom des Mediums vollständig in einem Abschnitt erfasste. Die Geschwindigkeit des Stroms betrug ca. 0,6 m/s und die Volumenwechselrate des bestrahlten Abschnitts betrug 100 Hz. Die erhaltenen Nanopartikel zeigten eine Größe von kleiner etwa 5 nm, eine mittlere Anzahlgrößenverteilung von etwa 2,5 nm und zeigten generell keine Resonanzfrequenz im grünen Bereich, insbesondere keine bei 520 nm, wie dies im Spektrum von Figur 2 gezeigt ist. Die Rotfärbung und Extinktion bei 520 nm der ersten Partikel verschwand vollständig, wenn das Medium wiederholt durch den zweiten Laserstrahl hindurchtrat, z.B. bis zu 60-mal.
In bevorzugter Ausführung wurden die Nanopartikel, die durch Bestrahlen des Stroms des Mediums mit darin suspendierten ersten Partikeln erzeugt wurden, zumindest noch einmal in einen freien Strom von Medium mit der zweiten gepulsten Laserstrahlung bestrahlt, beispielsweise durch Rückführen der in dem Medium suspendierten Nanopartikel durch die Düse, wiederum mit abschnittsweiser Bestrahlung des Stroms mit der zweiten gepulsten Laserstrahlung. Wie generell bevorzugt wurde die Repetitionsrate des Lasers an die Geschwindigkeit des Stroms angepasst, dass jedes Volumenelement des Stroms genau einmal mit einem Puls der Laserbestrahlung beaufschlagt wird.

Die erhaltenen Nanopartikel waren ohne weiteren Zusatz in dem Medium, das anorganisches Oxidationsmittel enthielt, als Suspension stabil, z.B. für zumindest 4 d, bevorzugt für zumindest 5 bis 30 d. Die Stabilität konnte auch daran gesehen werden, dass keine Plasmonresonanz auftrat, die ein Anzeichen für eine Aneinanderlagerung von nicht plasmonenresonanten Nanopartikeln ist.

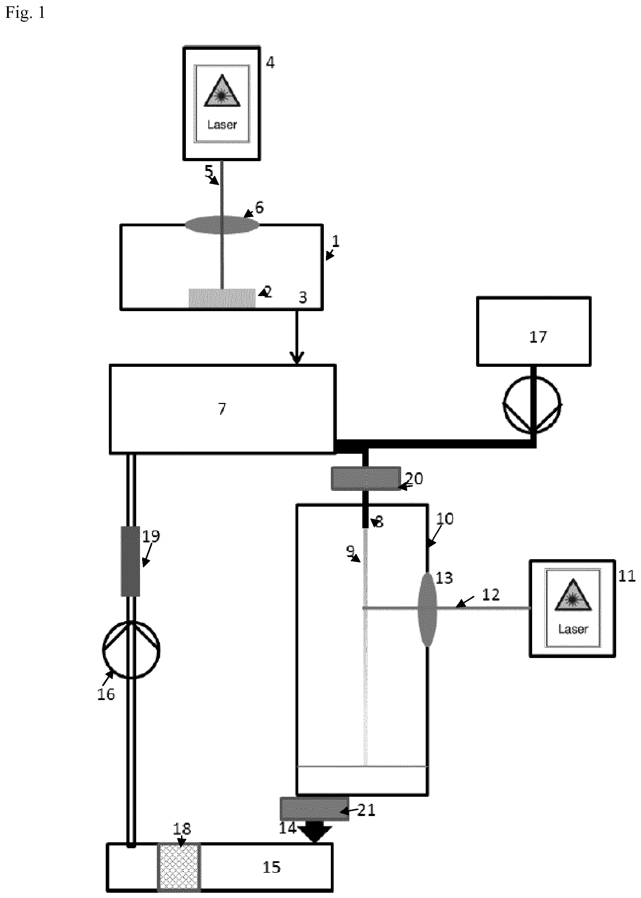

Die für dieses Verfahren eingesetzte Vorrichtung ist schematisch in der Figur 1 gezeigt. Dabei enthält ein Behälter 1 den Metallkörper 2 aus Gold auf einer Halteeinrichtung und ist von Medium 3 umgeben. Ein erster Laser 4 erzeugt die erste Laserstrahlung 5, die durch ein optisch transparentes Fenster 6 des Behälters 1 auf den Metallkörper 2 gerichtet ist. Bevorzugt ist der Behälter 1 eine von Medium 3 durchströmte Kammer mit einem Einlass und einem Auslass. Von dem Behälter 1 wird das Medium 3 im Anschluss an die Bestrahlung mit erster Laserstrahlung 5 in einen Vorratsbehälter 7 überführt, optional kontinuierlich. Optional ist in der Leitung zwischen dem Behälter 1 und dem Vorratsbehälter 7 und/oder in der Leitung zwischen dem Vorratsbehälter 7 und der Düse 8 ein Spektrometer 20 angeordnet, das zur Messung der Extinktion eingerichtet ist. Durch diese Anordnung ist das Spektrometer 20 in einer Leitung angeordnet, die vor der Düse 8 liegt und ist daher eingerichtet, das der Düse 8 zugeführte Medium zu messen. Bevorzugt ist ein Spektrometer 20 eingerichtet, eine Dosiereinrichtung für die Zugabe des anorganischen Oxidationsmittels zu steuern. Aus dem Vorratsbehälter 7 wird das Medium 3 mit den darin enthaltenen ersten Partikeln einer Düse 8 zugeleitet, die vertikal nach unten gerichtet ist und einen freien Mediumstrom 9 erzeugt. Zur Vermeidung von Aerosolbildung ist die Düse 8 innerhalb eines Gehäuses 10 angeordnet, in dem der Mediumstrom 9 frei fällt, optional durch eine Pumpe getrieben. Ein Abschnitt des Stroms des Mediums 9 wird von der von einem zweiten Laser 11 erzeugten zweiten Laserstrahlung 12 bestrahlt, die z.B. durch ein für die zweite Laserstrahlung 12 durchlässiges Fenster oder Loch 13 des Gehäuses 10 tritt. Wie schematisch angedeutet kann das Fenster 13 ein optisches Element, insbesondere eine Sammellinse aufweisen oder daraus bestehen.
Das Gehäuse 10 weist einen Auslass 14 auf, aus dem das Medium mit den nun darin enthaltenen Nanopartikeln austritt, beispielsweise in ein Sammelgefäß 15. Optional ist ein weiteres Spektrometer 21 am Auslass 14 angeordnet, das insbesondere mit einer Steuerungseinrichtung des zweiten Lasers 11 oder mit einer Steuerungseinrichtung der Pumpe, die den Mediumstrom 9 treibt, verbunden sein kann. Das Sammelgefäß 15 kann mit einer Rückführleitung 16 mit der Düse 8 verbunden sein, um eine wiederholte Bestrahlung des Mediums mit den nun darin enthaltenen Nanopartikeln und/oder ersten Partikeln durch zweite Laserstrahlung 12 zu erlauben. Im Sammelgefäß 15 kann ein Träger 18 angeordnet sein, wobei der Träger 18 insbesondere den Strömungsquerschnitt des Sammelgefäßes 18 überdeckt. Ein solcher Träger 18 ist z.B. im Sammelgefäß 15 angeordnet, um Nanopartikel auf dem Träger 18 anzuordnen. Der Träger 18 kann z.B. Titandioxid oder Aluminiumoxid, als Pulver oder Formkörper sein, auf den Nanopartikel sorbieren. In der Rückführleitung 16, die eine Pumpe aufweisen kann, ist optional ein Filter 19 enthalten, der Partikel mit einer Größe oberhalb einer vorgegebenen Größe zurückhält, z.B. Partikel mit einer Größe oberhalb 10 nm zurückhält. Eine Dosiereinrichtung für anorganisches Oxidationsmittel ist anhand eines Vorratsbehälters 17 für das Oxidationsmittel gezeigt, der mittels einer Leitung mit der Leitung verbunden ist, die zur Düse 8 führt. Auf diese Weise kann die Dosiereinrichtung, die vorzugsweise abhängig von dem am Auslass 14 angeordneten Spektrometer 21 gesteuert ist, eingerichtet sein, dem Medium, das erste Partikel enthält, anorganisches Oxidationsmittel zuzuführen.

Figur 2 zeigt ein Spektrum von erfindungsgemäß hergestellten Nanopartikeln aus Gold in H₂O₂ -haltigem Wasser im Vergleich zu Nanopartikeln aus Gold in reinem Wasser und daher ohne anorganisches Oxidationsmittel, die ansonsten mit dem gleichen Verfahren erzeugt wurden. Die Abwesenheit der Plasmonresonanz bei 520 nm erfindungsgemäßer Nanopartikel zeigt, dass die erfindungsgemäß in dem Medium mit einen Gehalt an anorganischen Oxidationsmittel hergestellten Nanopartikel kleiner als 5 nm, insbesondere kleiner als 3,5 nm im Durchmesser sind und sich nicht zu größeren, plasmonresonanten Aggregaten aneinander angelagert haben. Die erfindungsgemäßen Partikel zeigen dieselben optischen Eigenschaften auch nach einer Lagerung von z.B. 20 Tagen und belegen daher die Stabilität der Nanopartikel, die in dem Medium suspendiert sind. Als Grund für diese Stabilität wird gegenwärtig eine elektrostatische Stabilisierung der Nanopartikel angenommen. Daher kann die Suspension der Nanopartikel, die mit dem erfindungsgemäßen Verfahren erhältlich ist, auch als elektrostatisch stabilisiertes Kolloid bezeichnet werden.

Figur 3a zeigt die Partikelgrößen der erfindungsgemäß hergestellten Nanopartikel aus Gold in einer TEM-Aufnahme, die auf einem Gitter abgelagert und im Transmissionselektronenmikroskop (TEM) vermessen wurden. Diese Daten zeigen, dass die erfindungsgemäß hergestellten Nanopartikel eine mittlere Partikelgröße von ca. 3 nm bzw. einen Größenbereich von ca. 1-5 nm aufweisen können.

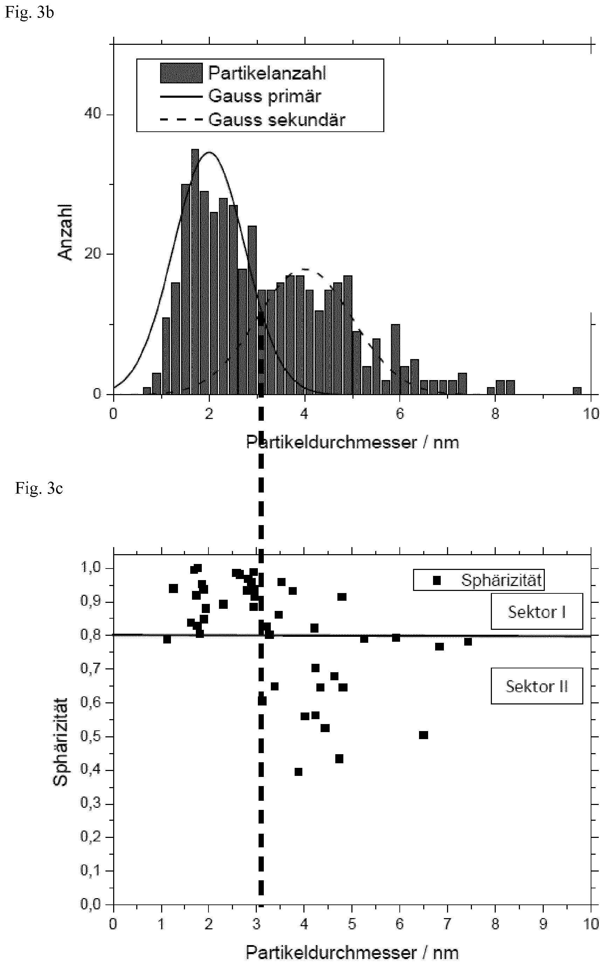

Figur 3b zeigt eine Größenverteilung erfindungsgemäßer Nanopartikel, die aus TEM-Aufnahmen bestimmt wurde. In der Größenverteilung sind eine primäre und eine sekundäre Gaußverteilung bestimmt. Die Partikelgröße, die im Schnittpunkt der Gaußverteilungen liegt, ist als Grenze bei der Darstellung der Sphärizität in Fig. 3c gezeigt. Für eine Sphärizität von unterhalb 0,8 (Sektor II) wird keine Kugelform angenommen.

Aus Figuren 3b und 3c ergibt sich, dass die kugelförmigen Partikel in Sektor I erfindungsgemäße Nanopartikel sind, die eine Partikelgröße von kleiner als 5 nm aufweisen, wobei ihr Hauptanteil eine Größe von kleiner 3 nm hat.

Aus den Daten der Figuren 3b und 3c kann geschlossen werden, dass größere Partikel möglicherweise erste Partikel sind oder als Artefakte bei der Präparation der Partikel auf dem TEM-Gitter entstanden sind, z.B. verursacht durch Entfernen des anorganischen Oxidationsmittels.

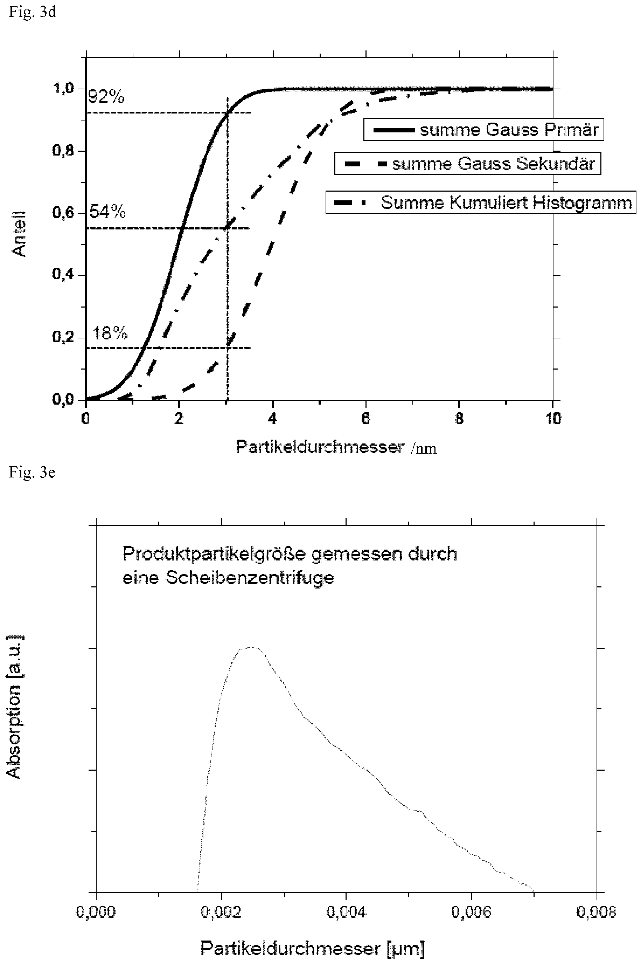

Figur 3d zeigt die Summenverteilung der einzelnen und kumulierten Gauß-Verteilungen. Hieraus wird ersichtlich, dass vorzugsweise 92% aller nach dem Verfahren erzeugen Partikel in dem Medium mit einem Durchmesser kleiner als 3 nm vorliegen.

Die in Figur 3e gezeigten Daten der Messung der Größe der Nanopartikel mittels einer Scheibenzentrifuge. Diese Daten bestätigen, dass erfindungsgemäß hergestellte Nanopartikel eine Größe von im Wesentlichen kleiner als 5 nm aufweisen, mit einer mittleren Größe von kleiner 3 bis kleiner 4 nm.

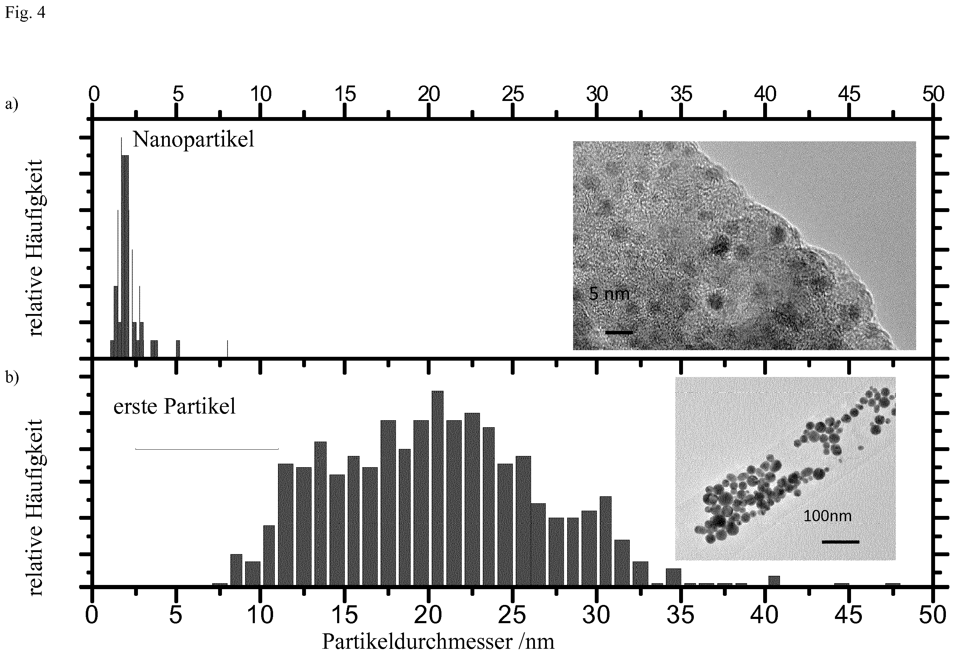

Figur 4 a) zeigt die Größenverteilung dieser erfindungsgemäß hergestellten Nanopartikel und eingesetzt ein elektronenmikroskopisches Bild dieser Nanopartikel. Figur 4 b) zeigt die Größenverteilung der mittels Laserbestrahlung des in Wasser mit 10 Gew.-% H₂O₂ angeordneten Goldkörpers erzeugten ersten Partikel. Das in Figur 4 b) eingesetzte elektronenmikroskopische Bild zeigt diese ersten Partikel. Alternativ kann das Wasser in diesem Schritt ohne Zusatz sein. Die ersten Partikel zeigen einen Durchmesser von ca. 7,5 bis maximal 50 nm, insbesondere von ca. 10 bis 35 nm, mit einer mittleren Partikelgröße von 20, z.B. für zumindest 90% der ersten Partikel.

Diese Ergebnisse zeigen, dass die erfindungsgemäß hergestellten Nanopartikel eine Größe von im Wesentlichen unter 5 nm, bevorzugt von 1 bis 3 nm, z.B. mit einer mittleren Größe von 2,5 nm aufweisen, generell bevorzugt mit einer monomodalen Größenverteilung.

### Beispiel 2: Herstellung einer Verbindung aus Nanopartikeln mit organischen Liganden

Nach Beispiel 1 erzeugte Nanopartikel aus Gold in einem Medium mit einem Gehalt an H₂O₂ wurden zunächst in ein nicht oxidierendes Medium überführt. Anschließend wurde die Suspension der Nanopartikel mit einem organischen Liganden als Beispiel für eine Substanz kontaktiert, beispielsweise mit einem Oligonukleotid, einem Protein, bevorzugt einem Bindemolekül, insbesondere einem Antikörper, oder mit einem Polysaccharid. Optional wies die Substanz eine Thiolgruppe auf.

Es hat sich gezeigt, dass die erfindungsgemäß erhältlichen Nanopartikel eine ausreichende Reaktivität aufweisen, um mit dem als Substanz zugesetzten organischen Molekül eine Bindung einzugehen. Die organischen Moleküle waren daher durch die Nanopartikel markiert. Die erhaltenen Verbindungen aus der Substanz mit erfindungsgemäß hergestellten Nanopartikeln waren fluoreszent und nicht plasmonresonant, wie dies generell bevorzugt ist.

### Beispiel 3: Beschichten eines anorganischen Trägers mit Nanopartikeln

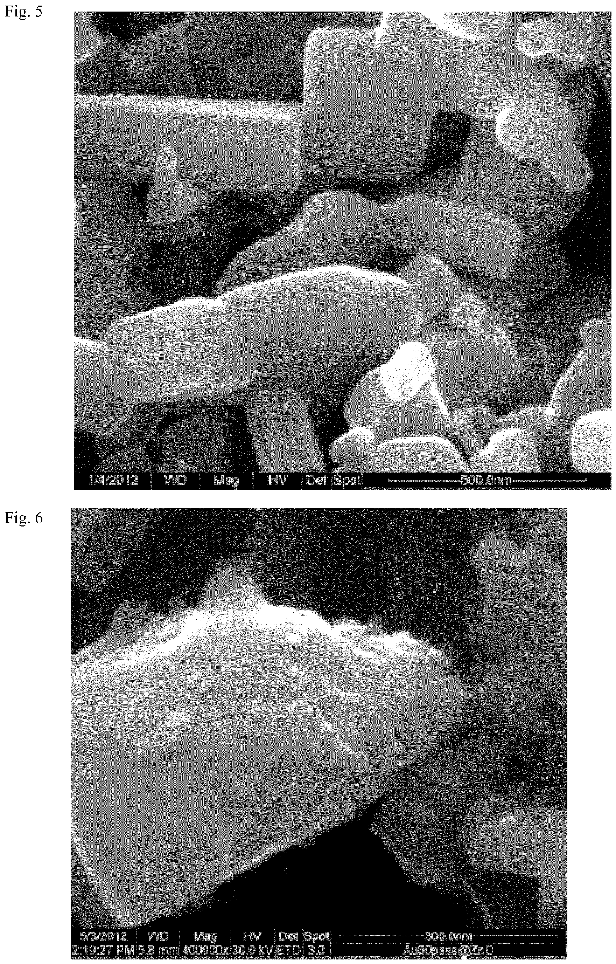

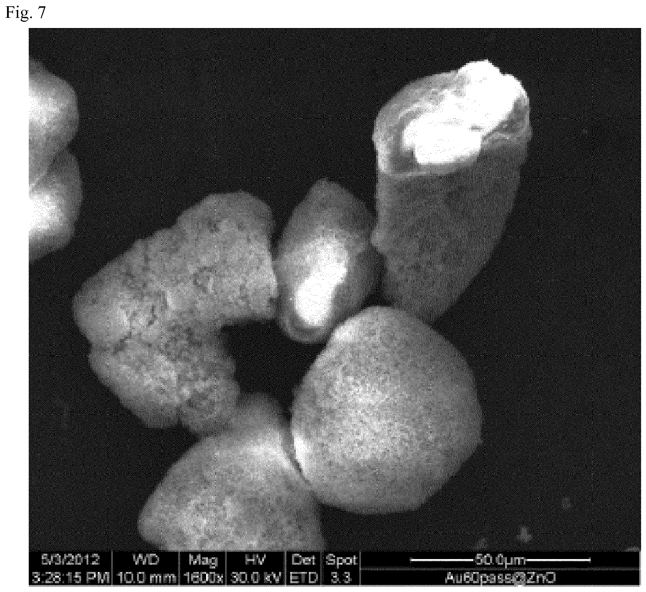

Als Beispiel für einen anorganischen Träger wurde Zinkoxid eingesetzt oder eine Elektrode mit metallischer Oberfläche. Die Nanopartikel wurden durch Kontaktierung mit dem Medium, das die Nanopartikel enthielt, auf dem Träger abgeschieden. Dabei zeigte sich, dass kein äußeres oder zusätzliches elektrisches Feld zur Anordnung der Nanopartikel auf dem Träger erforderlich war. Die Nanopartikel konnten alternativ durch elektrophoretische Abscheidung oberflächlich auf den Träger, der bevorzugt Oberflächenladungen aufwies, abgeschieden werden und bildeten eine adsorbierte Schicht. Das Zinkoxid, das mit den Nanopartikeln aus Gold beschichtet war, zeichnete sich durch eine homogene Anordnung bzw. Schicht aus sorbierten Nanopartikeln aus. Figur 5 zeigt die als Träger eingesetzten Zinkoxidpartikel in einer rasterelektronenmikroskopischen (REM) Aufnahme, die Figuren 6 und 7 zeigen die Zinkoxidpartikel nach Kontaktierung mit den suspendierten Nanopartikeln. Hier wird deutlich, dass diese Nanopartikel auf der Oberfläche des Trägers sorbieren.

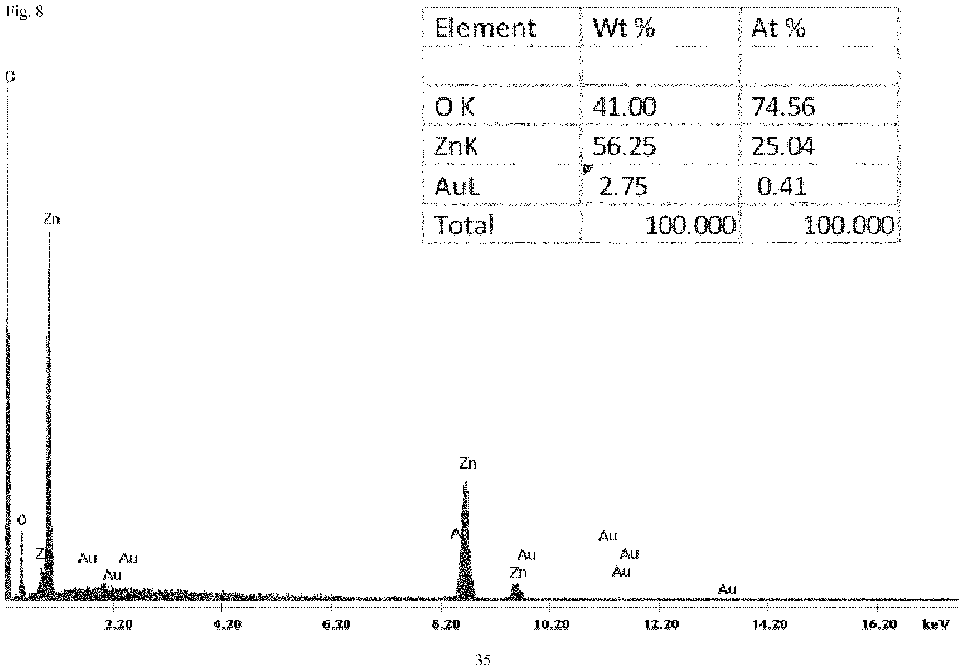

Figur 8 zeigt eine EDX-Analyse der erfindungsgemäßen Nanopartikel aus Gold, die auf ZnO-Träger angeordnet sind. Sauerstoff ist anhand der K-Linie (O K), Zink anhand der K-Linie (ZnK) und Gold anhand der L-Linie (AuL) des Spektrums bestimmt worden.

Aus der EDX-Analyse wird deutlich, dass die Nanopartikel aus Gold, die nichtplasmonresonant sind und die bevorzugt Cluster sind, auf den Träger sorbiert sind und eine nicht-plasmonresonante Schicht bilden.

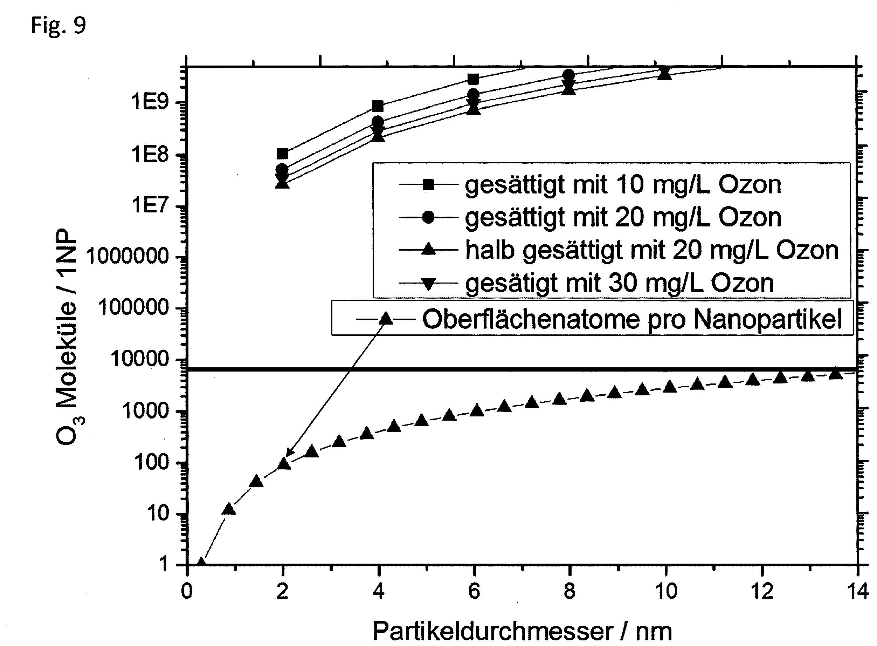

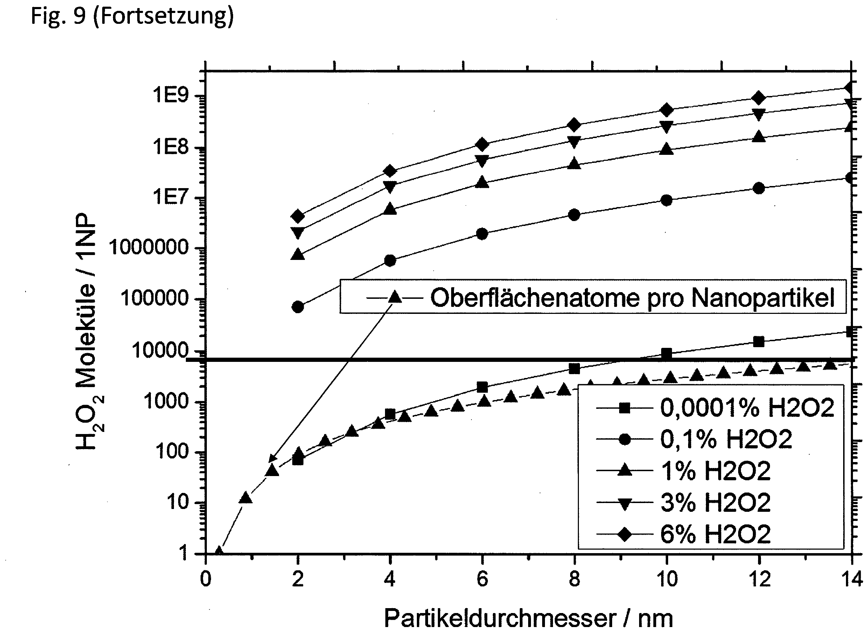

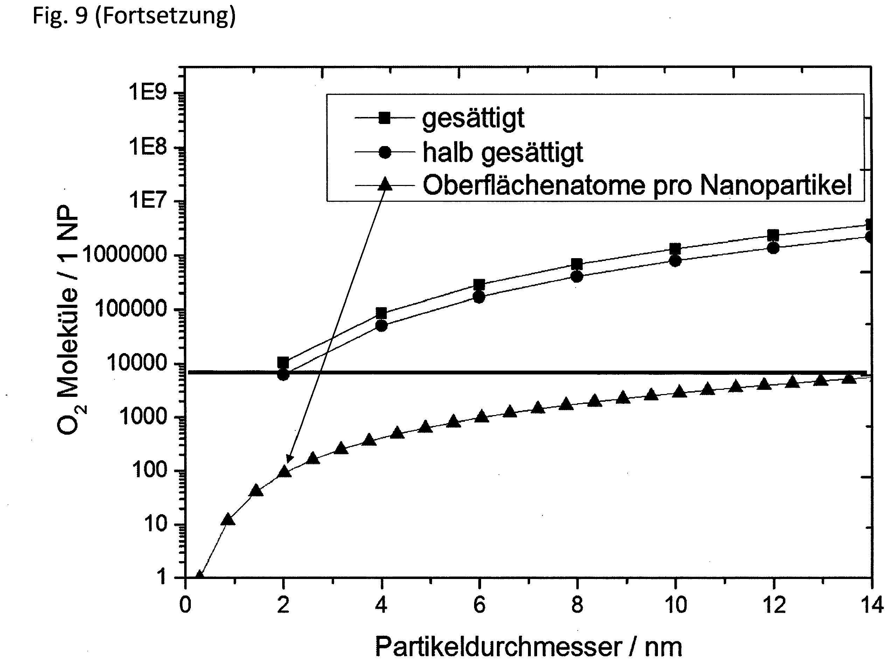

Figur 9 zeigt die berechneten molaren Konzentrationen der Oxidationsmittel Ozon, H₂O₂ (erfindungsgemäß) und Sauerstoff, jeweils bei Normaldruck unter Standardbedingungen in Wasser (nicht erfindungsgemäß) sowie die für eine Goldnanopartikelgröße von 2nm bei einer Konzentration von 20mg/L berechnete molare Konzentration von Nanopartikeln. Es zeigt sich, dass für H₂O₂ eine Konzentration von lGew.-% bereits eine um einen Faktor von 1000 höheres molares Verhältnis zur molaren Konzentration der Nanopartikel ergibt, als die Sättigung mit Sauerstoff.

Am Beispiel von Goldnanopartikeln ist die Anzahl der Oberflächenatome der Nanopartikel für die angegebenen Partikelgrößen angegeben. Dies macht deutlich, dass mit zunehmender Größe der Nanopartikel die Anzahl der Oberflächenatome je Nanopartikel zunimmt, allerdings nicht linear.

### Beispiel 4: Herstellung eines optischen Elements

Als Beispiel für ein optisches Element wurde ein Glas mit den in dem Medium suspendierten Nanopartikeln kontaktiert. Das Medium konnte den Gehalt an anorganischen Oxidationsmittel aufweisen, alternativ konnte das anorganische Oxidationsmittel aus dem Medium entfernt werden.

Die erfindungsgemäß erhältlichen Nanopartikel konnten durch bloßes Kontaktieren des Glases mit der Suspension darauf abgeschieden werden.

Das so hergestellte optische Element zeigte bei Bestrahlung, insbesondere in einem Wellenlängenbereich von 350 bis 1064 nm eine limitierbare Lichtundurchlässigkeit, die die Eignung der erfindungsgemäß erhältlichen Nanopartikel zur Verwendung als optische Begrenzer gegen Strahlung dieser Wellenlänge zeigen.

## Patentansprüche

1. Verfahren zur Herstellung von Nanopartikeln aus Gold und/oder einem Metall der Platingruppe durch Bereitstellen erster Partikel aus Gold und/oder dem Platinmetall, die in einem wässrigen Medium suspendiert sind, wobei dem wässrigen Medium ein anorganisches Oxidationsmittel zugesetzt ist, **gekennzeichnet dadurch, dass** die in dem wässrigen Medium suspendierten ersten Partikel durch Aufbringen einer ersten gepulsten Laserstrahlung auf einen in einem wässrigen Medium angeordneten metallhaltigen Körper, der Gold und/oder ein Metall der Platingruppe enthält, oder durch Drahterosion eines metallhaltigen Körpers in Form eines Drahts aus Gold und/oder einem Metall der Platingruppe in dem wässrigen Medium erzeugt werden, wobei das wässrige Medium frei von organischen Kohlenstoffverbindungen ist, dass das Medium mit den darin suspendierten ersten Partikeln mit einer gepulsten Laserstrahlung bestrahlt wird und dass das anorganische Oxidationsmittel in dem wässrigen Medium ein größeres Redoxpotential aufweist als es eine oxidierte Form von Gold und/oder des Metalls der Platingruppe aufweist, die durch die Laserstrahlung entsteht, , wodurch eine Suspension der Nanopartikel in dem Medium erzeugt wird.

2. Verfahren nach Anspruch 1, **dadurch gekennzeichnet, dass** das anorganische Oxidationsmittel in dem wässrigen Medium ein größeres Redoxpotential hat, als das Redoxpotential, das die Nanopartikel einer oxidierten Form von Gold und/oder des Platinmetalls in dem wässrigen Medium aufweisen.

3. Verfahren nach einem der voranstehenden Ansprüche, **dadurch gekennzeichnet, dass** das wässrige Medium und die darin suspendierten ersten Partikel von organischen Kohlenstoffverbindungen frei sind und/oder dass die ersten Partikel eine Größe von maximal 200nm aufweisen.

4. Verfahren nach einem der voranstehenden Ansprüche, **gekennzeichnet durch** das Erzeugen eines Stroms aus dem wässrigen Medium, dem das anorganische Oxidationsmittel zugesetzt ist, mit darin suspendierten ersten Partikeln, wobei die gepulste Laserstrahlung auf zumindest einen Abschnitts des Stroms gerichtet ist.

5. Verfahren nach Anspruch 4, **dadurch gekennzeichnet, dass** der Strom des Mediums mit den darin suspendierten ersten Partikeln ein freier Flüssigkeitsstrom ist.

6. Verfahren nach einem der Ansprüche 4 bis 5, **dadurch gekennzeichnet, dass** der Durchmesser des Abschnitts des Stroms maximal so groß ist wie der auf diesen Abschnitt gerichtete Fokus der gepulsten Laserstrahlung.

7. Verfahren nach einem der voranstehenden Ansprüche, **dadurch gekennzeichnet, dass** die gepulste Laserstrahlung eine Wellenlänge von 330 bis 1500 nm bei einer Repetitionsrate von mindestens 10 Hz aufweist.

8. Verfahren nach einem der voranstehenden Ansprüche, **dadurch gekennzeichnet, dass** die gepulste Laserstrahlung eine Fluenz von zumindest 0,8 J/cm² aufweist.

9. Verfahren nach einem der voranstehenden Ansprüche, **dadurch gekennzeichnet, dass** das Medium, in dem die ersten Partikel suspendiert sind, aus Wasser mit einem darin enthaltenen anorganischen Oxidationsmittel besteht und das Oxidationsmittel ausgewählt ist aus der Gruppe, die aus H₂O₂, gelöstem Ozon, einem Derivat aus Sauerwasserstoff, einem Stickoxid, Antimonsäure, arseniger Säure, Arsensäure, Borsäure, chloriger Säure, bromiger Säure, Chlorsäure, Chromsäure, Cyansäure, Dichromsäure, Dischwefelsäure, hypochloriger Säure, hypobromiger Säure, hypoiodiger Säure, iodiger Säure, Iodsäure, Isocyansäure, Kohlensäure, Metakieselsäure, Molybdänsäure, Orthodikieselsäure, Orthokieselsäure, Perbromsäure, Perchlorsäure, Periodsäure (Orthoperiodsäure), Peroxodischwefelsäure, Peroxosalpetersäure, Phosphorsäure, Salpetersäure, salpetriger Säure, Schwefelsäure, schwefliger Säure, Tellursäure, Thioschwefelsäure, Wolframsäure oder deren Salzen, hypofluoriger Säure, Hypofluorit, Chlorsauerstoffsäure, Hypochlorit, Chlorit, Chlorat, Perchlorat, Bromsauerstoffsäure, Hypobromit, bromiger Säure, Bromit, Bromsäure, Bromat, Perbromsäure, Perbromat, Iodsauerstoffsäure, Hypoiodit, Iodit, Iodsäure, Iodat, ortho-Periodsäure, Periodat, meta-Periodsäure und Mischungen dieser besteht, wobei für Oxidationsmittel, die in dem wässrigen Medium gelöste Gase sind, deren Redoxpotential zumindest um deren Überspannung gegenüber den Nanopartikeln größer ist.

10. Verfahren nach einem der voranstehenden Ansprüche, **dadurch gekennzeichnet, dass** die in dem Medium suspendierten Nanopartikel anschließend, optional nach Entfernen des anorganischen Oxidationsmittels, mit einer Substanz kontaktiert werden, die ausgewählt ist aus organischen Liganden, einem anorganischen Träger und einem optisch aktiven Festkörper.

11. Verfahren nach einem der Ansprüche 1 bis 10, **dadurch gekennzeichnet, dass** der metallhaltige Körper ein reines Metall oder eine Metalllegierung von zumindest zwei Metallen der Platingruppe ist.

12. Verfahren nach einem der voranstehenden Ansprüche, **dadurch gekennzeichnet, dass** die Nanopartikel Atomcluster sind.

13. Suspension metallhaltiger Nanopartikel, erhältlich durch ein Verfahren nach einem der voranstehenden Ansprüche, **dadurch gekennzeichnet, dass** die Nanopartikel in Suspension in Medium vorliegen, das Wasser ist und das frei von organischen Kohlenstoffverbindungen ist und einen Gehalt an einem anorganischen Oxidationsmittel aufweist, das in dem wässrigen Medium ein größeres Redoxpotential aufweist als es eine oxidierte Form von Gold und/oder des Metalls der Platingruppe aufweist, die durch die Laserstrahlung entsteht, wobei die Nanopartikel keinen organischen Liganden aufweisen, die Nanopartikel eine maximale Größe von 5 nm aufweisen und eine mittlere Größe von 2 bis 3 nm, die zumindest 50% der Nanopartikel aufweisen, wobei die Größe im Transmissionselektronenmikroskop (TEM) oder mittels einer Scheibenzentrifuge vermessen ist.

14. Vorrichtung zur Herstellung von Nanopartikeln zur Verwendung in einem Verfahren nach einem der Ansprüche 1 bis 12, mit einem Behälter (1), in dem eine Halterung zur Fixierung eines Metallkörpers (2) angeordnet ist, wobei der Behälter (1) zur Aufnahme eines Mediums (3) eingerichtet ist und ein erster Laser (4) eingerichtet ist, den in der Halterung angeordneten Metallkörper (2) mit erster Laserstrahlung (5) zu bestrahlen, **gekennzeichnet durch** eine Einrichtung zur Zuführung von Medium (3) aus dem Behälter (1) zu einer Düse (8), die zur Erzeugung eines Stroms aus dem Medium (3) mit darin enthaltenen ersten Partikeln eingerichtet ist und mit einem zweiten Laser (11), der zur Einstrahlung einer zweiten Laserstrahlung (12) auf einen Abschnitt des aus der Düse (8) austretenden Stroms (9) eingerichtet ist.

15. Vorrichtung nach Anspruch 14, **gekennzeichnet durch** eine Rückführleitung (16), die eingerichtet ist, den aus der Düse (8) austretenden Strom (9) aus Medium mit darin enthaltenen Nanopartikeln im Anschluss an den Durchtritt durch die zweite Laserstrahlung (12) zur Düse (8) zurückzuführen.

16. Vorrichtung nach Anspruch 14 oder 15, **gekennzeichnet durch** eine Dosiereinrichtung zur Dosierung eines anorganischen Oxidationsmittels in eine Leitung oder in einen Behälter (1), der vor der Düse (8) angeordnet ist und durch ein in einer Leitung vor der Düse (8) angeordnetes Spektrometer (20) oder durch ein nach dem Auslass (14) des Gehäuses (10) angeordnetes Spektrometer (21), wobei die Dosiereinrichtung eingerichtet ist, abhängig vom Spektrometer (20, 21) gesteuert zu werden.

## Claims

1. Process for producing nanoparticles of gold and/or of a metal of the platinum group by providing first particles of gold and/or of the platinum metal, which are suspended in an aqueous medium, wherein an inorganic oxidation agent is added to the aqueous medium, **characterized in that** the first particles suspended in the aqueous medium are generated by applying a first pulsed laser irradiation onto a metal containing body which contains gold and/or a metal of the platinum group arranged in an aqueous medium, or by wire erosion of a metal containing body in the form of a wire of gold and/or of the metal of the platinum group in the aqueous medium, wherein the aqueous medium is free from organic carbon compounds, that the medium with the first particles suspended therein is irradiated by a pulsed laser irradiation and that the inorganic oxidation agent in the aqueous medium has a higher redox potential than an oxidized form of gold and/or of the metal of the platinum group which is produced by the laser irradiation, whereby a suspension of the nanoparticles in the medium is generated.

2. Process according to claim 1, **characterized in that** the inorganic oxidation agent in the aqueous medium has a higher redox potential than the redox potential of the nanoparticles of an oxidized form of gold and/or of the platinum metal in the aqueous medium.

3. Process according to one of the preceding claims, **characterized in that** the aqueous medium and the first particles suspended therein are free from organic carbon compounds and/or that the first particles have a size of at maximum 200 nm.

4. Process according to one of the preceding claims, **characterized by** the generation of a stream of the aqueous medium, to which the inorganic oxidation agent is added, with the first particles suspended therein, wherein the pulsed laser irradiation is directed onto at least a section of the stream.

5. Process according to claim 4, **characterized in that** the stream of the medium with the first particles suspended therein is a free liquid stream.

6. Process according to one of claims 4 to 5, **characterized in that** the diameter of the section of the stream is at maximum as large as the focus of the pulsed laser irradiation directed onto the section.

7. Process according to one of the preceding claims, **characterized in that** the pulsed laser irradiation has a wavelength of 330 to 1500 nm at a repetition rate of at least 10 Hz

8. Process according to one of the preceding claims, **characterized in that** the pulsed laser irradiation has a fluency of at least 0.8 J/cm².

9. Process according to one of the preceding claims, **characterized in that** the medium, in which the first particles are suspended, consists of water with an inorganic oxidation agent contained therein, and the oxidation agent is selected from the group consisting of H₂O₂, dissolved ozon, a derivative of oxygen - hydrogen compounds, a nitrogen oxide, antimonic acid, arsenious acid, arsenic acid, boric acid, chlorous acid, bromous acid, chloric acid, chromic acid, cyanic acid, dichromic acid, disulphuric acid, hypochlorous acid, hypobromous acid, hypoiodous acid, iodous acid, iodic acid, isocyanic acid, carbonic acid, metasilicilic acid, molybdic acid, orthodisilicilic acid, orthosilicilic acid, perbromic acid, perchloric acid, periodic acid (orthoperiodic acid), peroxodisulphuric acid, peroxonitric acid, phosphoric acid, nitric acid, nitrous acid, sulphuric acid, sulphurous acid, telluric acid, thiosulphuric acid, tungstic acid and its salts, hypofluorous acid, hypofluorite, oxyacid of chlorine, hypochlorite, chlorite, chlorate, perchlorate, oxyacid of bromine, hypobromite, bromous acid, bromite, bromic acid, bromate, perbromic acid, perbromate, oxyacids of iodine, hypoiodite, iodite, iodic acid, iodate, orthoperiodic acid, periodate, meta-periodic acid and admixtures of these, wherein for oxidizing agents which are gases dissolved in the aqueous medium their redox potential is higher by at least their overpotential over the nanoparticles.

10. Process according to claim 1, **characterized in that** the nanoparticles suspended in the medium are subsequently, optionally following removal of the inorganic oxidizing agent, contacted with a substance which is selected from organic ligands, an inorganic carrier and an optically active solid.

11. Process according to claims 1 to 10, **characterized in that** the metal containing body is a pure metal or a metal alloy of at least two metals of the platinum group.

12. Process according to one of the preceding claims, **characterized in that** the nanoparticles are atomic clusters.

13. Suspension of metal containing nanoparticles, obtainable by a process according to one of the preceding claims, **characterized in that** the nanoparticles are present in suspension in a medium, which is water and which is free from organic carbon compounds and has a content of an inorganic oxidation agent which in the aqueous medium has a higher redox potential than an oxidized form of gold and/or of the metal of the platinum group which is generated by the laser irradiation, wherein the nanoparticles have no organic ligand, the nanoparticles have a maximum size of 5 nm and a medium size of 2 to 3 nm, which are present in at least 50% of the nanoparticles, wherein the size is measured in a transmission electron microscope (TEM) or by means of a disk centrifuge.

14. Device for producing nanoparticles for use in a process according to one of claims 1 to 12 having a container (1) in which a mounting for fixation of a metal body (2) is arranged, wherein the container (1) is disposed for receiving a medium (3) and a first laser (4) is disposed to irradiate the metal body (2) arranged in the mounting by a first laser irradiation (5), **characterized by** a device for introducing a medium (3) from the container (1) to a nozzle (8) which is disposed for generating a flow of the medium (3) having first particles contained therein and with a second laser (11) which is disposed for irradiating a second laser irradiation (12) onto a section of the flow (9) exiting from the nozzle (8).

15. Device according to claim 14, **characterized by** a return duct (16) that is disposed to recirculate the flow (9) of medium to the nozzle (8), the medium having the nanoparticles contained therein exiting from the nozzle (8) subsequent to the passage through the second laser irradiation (12) to the nozzle (8).

16. Device according to claims 14 or 15, **characterized by** a dosing device for dosing an inorganic oxidizing agent into a duct or into a container (1) which is arranged upstream of the nozzle (8) and by a spectrometer (20) arranged in a duct upstream of the nozzle (8) and by a spectrometer (21) arranged downstream from the outlet (14) of the housing (10), wherein the dosing device is disposed to be controlled in dependence from the spectrometer (20, 21).

## Revendications

1. Procédé de production de nanoparticules d'or et/ou d'un métal du groupe du platine en fournissant des premières particules d'or et/ou du métal de platine en suspension dans un milieu aqueux, dans lequel un agent oxydant inorganique est ajouté au milieu aqueux, **caractérisé en ce que** les premières particules en suspension dans le milieu aqueux sont obtenues en appliquant un premier rayonnement laser pulsé sur un corps contenant du métal disposé dans un milieu aqueux, qui contient de l'or et/ou un métal du groupe du platine, ou par érosion par fil d'un corps contenant un métal sous la forme d'un fil d'or et/ou d'un métal du groupe du platine dans le milieu aqueux,
dans lequel le milieu aqueux est exempt de composés carbonés organiques, que le milieu avec les premières particules en suspension est irradié avec un rayonnement laser pulsé et **en ce que** l'agent oxydant inorganique a un potentiel redox supérieur dans le milieu aqueux par rapport à une forme oxydée de l'or et/ou du métal du groupe du platine, forme générée par le rayonnement laser, une suspension des nanoparticules étant générée dans le milieu.

2. Procédé selon la revendication 1, **caractérisé en ce que** l'agent oxydant inorganique dans le milieu aqueux présente un potentiel redox supérieur au potentiel redox présenté par les nanoparticules d'une forme oxydée de l'or et/ou du platine dans le milieu aqueux.

3. Procédé selon l'une des revendications précédentes, **caractérisé en ce que** le milieu aqueux et les premières particules en suspension dans celui-ci sont exempts de composés carbonés organiques et/ou que les premières particules ont une taille d'au plus 200 nm.

4. Procédé selon l'une des revendications précédentes, **caractérisé par** la génération d'un écoulement de milieu aqueux auquel l'agent oxydant inorganique est ajouté, les premières particules étant en suspension dans celui-ci, le rayonnement laser pulsé étant dirigé sur au moins une partie du courant.

5. Procédé selon la revendication 4, **caractérisé en ce que** l'écoulement du milieu avec les premières particules en suspension est un écoulement de liquide libre.

6. Procédé selon l'une des revendications 4 ou 5, **caractérisé en ce que** le diamètre de la partie du courant est au plus grand que le foyer dirigé sur cette section du rayonnement laser pulsé.

7. Procédé selon l'une des revendications précédentes, **caractérisé en ce que** le rayonnement laser pulsé a une longueur d'onde de 330 à 1500 nm à un taux de répétition d'au moins 10 Hz.

8. Procédé selon l'une des revendications précédentes, **caractérisé en ce que** le rayonnement laser pulsé a une fluence d'au moins 0,8 J/cm².

9. Procédé selon l'une des revendications précédentes, **caractérisé en ce que** le milieu dans lequel sont suspendues les premières particules est constitué d'eau avec un oxydant inorganique contenu dans celui-ci et l'agent oxydant est choisi dans le groupe constitué de H₂O₂, d'ozone dissous, d'un dérivé d'hydrogène acide, d'oxyde nitrique, d'acide antimonique, d'acide arsénieux, d'acide arsénique, d'acide borique, d'acide chloreux, d'acide bromé, d'acide chlorique, d'acide chromique, d'acide cyanique, d'acide dichromique, d'acide disulfurique, d'acide hypochloreux, d'acide hypobromeux, d'acide hypoiodeux, d'acide iodique, d'acide d'iode, d'acide isocyanique, d'acide carbonique, d'acide métasilicique, d'acide molybdique, d'acide orthodisilicique, d'acide orthosilicique, d'acide pérbromique, d'acide perchlorique, d'acide périodique (d'acide orthopériodique), d'acide peroxodisulfurique, d'acide péroxonitrique, d'acide phosphorique, d'acide nitrique, d'acide nitreux, d'acide sulfurique, d'acide sulfureux, d'acide tellurique, d'acide thiosulfurique, d'acide tungstique ou leurs sels, d'acide hypofluorique, d'hypofluorite, d'acide chlorosaurique, d'hypochlorite, de chlorite, de chlorate, de perchlorate, d'acide bromosurique, d'hypobromite, d'acide bromeux, de bromite, d'acide bromique, de bromate, d'acide perbromique, de perbromate, d'acide iodique, d'hypoiodite, d'iodite, d'acide iodique, de iodate, d'acide orthopériodique, de periodate, d'acide métapériodique et les mélanges de ceux-ci,
dans lequel, pour les agents oxydants qui sont des gaz dissous dans le milieu aqueux, leur potentiel redox est au moins supérieur par leur surtension par rapport aux nanoparticules.

10. Procédé selon l'une des revendications précédentes, **caractérisé en ce que** les nanoparticules en suspension dans le milieu sont ensuite mises en contact, éventuellement après élimination de l'agent oxydant inorganique, avec une substance choisie parmi les ligands organiques, un support inorganique et un solide optiquement actif.

11. Procédé selon l'une des revendications 1 à 10, **caractérisé en ce que** le corps contenant un métal est un métal pur ou un alliage métallique d'au moins deux métaux du groupe du platine.

12. Procédé selon l'une des revendications précédentes, **caractérisé en ce que** les nanoparticules sont des amas atomiques.

13. Suspension de nanoparticules contenant un métal, susceptible d'être obtenue par un procédé selon l'une quelconque des revendications précédentes, **caractérisée en ce que** les nanoparticules sont en suspension dans un milieu qui est de l'eau et exempt de composés organiques carbonés et qui contient un agent oxydant inorganique, que le milieu aqueux a un potentiel redox supérieur par rapport à une forme oxydée de l'or et/ou du métal du groupe du platine, forme générée par le rayonnement laser, tandis que les nanoparticules n'ont pas de ligand organique, les nanoparticules ont une taille maximale de 5 nm et une taille moyenne de 2 à 3 nm, qui comprennent au moins 50% des nanoparticules, la taille étant mesurée par microscopie électronique à transmission (MET) ou au moyen d'une centrifugeuse à disque.

14. Dispositif de fabrication de nanoparticules utilisables dans un procédé selon l'une des revendications 1 à 12, comprenant un récipient (1) dans lequel est agencé un support de fixation d'un corps métallique (2), dans lequel le récipient (1) est conçu pour recevoir un support (3) et un premier laser (4) est agencé pour irradier le corps métallique (2) disposé dans le support avec un premier rayonnement laser (5), **caractérisé par** des moyens pour amener le milieu (3) du récipient (1) à une buse (8) conçue pour générer un courant à partir du milieu (3) contenant des premières particules et un second laser (11) agencé pour irradier un second rayonnement laser (12) sur une partie du flux (9) sortant de la buse (8).

15. Appareil selon la revendication 14, **caractérisé par** une conduite de retour (16) qui est agencée pour ramener à la buse (8) le flux sortant (9) de la buse (8) à partir du milieu, lequel flux contient des nanoparticules, après avoir pénétré dans le second rayonnement laser (12).

16. Appareil selon les revendications 14 ou 15, **caractérisé par** un dispositif de dosage destiné à doser un agent oxydant inorganique dans un conduit ou dans un récipient (1) disposé devant la buse (8) et par un spectromètre (20) disposé dans une conduite devant la buse (8) ou par un spectromètre (21) disposé après la sortie (14) du boîtier (10), dans lequel le dispositif de dosage est agencé pour être commandé en fonction du spectromètre (20, 21).
